# FASCICULE DE BREVET EUROPEEN

(11) **EP 3 860 459 B1**
(45) Date de publication et mention de la délivrance du brevet: **30.03.2022**
(21) Numéro de dépôt: 19783302.3
(22) Date de dépôt: 04.10.2019
(51) Int. Cl.: A61B 5/11, A61B 5/22, A61B 5/00

(54) **DISPOSITIF DE QUANTIFICATION DE LA DEXTERITE**
VORRICHTUNG ZUR QUANTIFIZIERUNG VON GESCHICKLICHKEIT
DEVICE FOR QUANTIFYING DEXTERITY

(30) Priorité: 04.10.2018 FR 1859202
(43) Date de publication de la demande: 11.08.2021
(73) Titulaire: INSTITUT NATIONAL DE LA SANTE ET DE LA RECHERCHE MEDICALE - INSERM, 75013 Paris (FR); CENTRE NATIONAL DE LA RECHERCHE SCIENTIFIQUE (CNRS), 75016 Paris (FR); Université de Paris, 75006 Paris (FR); SENSIX, 86000 Poitiers (FR)
(72) Inventeur: TEREMETZ, Maxime, 75013 PARIS (FR); BOUCHER, Mathieu, 86000 Poitiers (FR); LINDBERG, Pavel, 75011 PARIS (FR); MAIER, Marc, 75010 PARIS (FR)
(74) Mandataire: A.P.I. Conseil
(86) Numéro de dépôt international: PCT/EP2019/076950
(87) Numéro de publication internationale: WO 2020/070305

(56) Documents cités:
- EP-A1- 2 659 835
- CN-B- 103 239 242
- US-A- 5 147 256
- US-A1- 2015 190 675
- US-B1- 9 597 547
- OLANDERSSON S ET AL: "Finger-Force Measurement-Device for Hand Rehabilitation", REHABILITATION ROBOTICS, 2005. ICORR 2005. 9TH INTERNATIONAL CONFERENC E ON CHICAGO, IL, USA JUNE 28-JULY 1, 2005, PISCATAWAY, NJ, USA,IEEE, 28 juin 2005 (2005-06-28), pages 135-138, XP010830407, DOI: 10.1109/ICORR.2005.1501069 ISBN: 978-0-7803-9003-4
- MILLER L C ET AL: "A Wrist and Finger Force Sensor Module for Use During Movements of the Upper Limb in Chronic Hemiparetic Stroke", IEEE TRANSACTIONS ON BIOMEDICAL ENGINEERING, IEEE SERVICE CENTER, PISCATAWAY, NJ, USA, vol. 56, no. 9, 1 septembre 2009 (2009-09-01), pages 2312-2317, XP011343045, ISSN: 0018-9294, DOI: 10.1109/TBME.2009.2026057

## Description

### DOMAINE DE L'INVENTION

L'invention concerne un dispositif pour quantifier la dextérité des doigts d'une main, par exemple en détectant le mouvement et/ou la force des doigts. Le dispositif peut être par exemple utilisé dans le cadre du diagnostic ou de la thérapie d'un accident vasculaire cérébral, ou dans les pathologies neurologiques, myologiques ou arthrologiques affectant la main.

### ETAT DE LA TECHNIQUE

L'altération de la dextérité manuelle est un problème majeur de santé publique puisqu'elle entraîne une altération des activités de la vie quotidienne et une perte d'autonomie. De nombreuses affections neurologiques, orthopédiques et rhumatologiques affectent la dextérité manuelle. Les affections neurologiques comprennent par exemple les accidents vasculaires cérébraux, la paralysie cérébrale, la sclérose en plaques, la spondylose cervicale, la myélopathie, le syndrome du canal carpien et la dystonie.

La dextérité manuelle peut être quantifiée en vue d'un diagnostic et/ou d'un pronostic clinique, ou d'une éventuelle rééducation des doigts.

A cet effet, EP 2 659 835 décrit un dispositif permettant de mesurer et quantifier le déplacement des doigts et la force exercée par les doigts sur le dispositif. Le dispositif comprend une pluralité de pistons insérés dans un corps principal. Chaque piston comprend un tube et un axe, le tube entourant l'axe du piston. Chaque tube est également relié à l'axe par un moyen de rappel, de sorte que l'axe exerce une force sur le capteur de force lors du mouvement du tube.

Toutefois, le dispositif décrit présente les inconvénients suivants :
- le dispositif ne comporte pas un piston pour le pouce
- le doigt d'un patient peut être instable une fois en contact avec la tête d'un piston, entraînant des erreurs de mesure du mouvement ou de la force du doigt,
- la mesure de la force ou du mouvement des doigts est limitée à quatre doigts de la main simultanément,
- seule la flexion d'un doigt peut être mesurée par le dispositif décrit.

### RESUME DE L'INVENTION

Un but de l'invention est de proposer un dispositif remédiant au moins partiellement aux inconvénients précités de l'art antérieur.

Ce but est atteint dans le cadre de la présente invention grâce à un dispositif pour quantifier la dextérité des doigts d'une main, comprenant :
- un corps principal, les dimensions du corps principal permettant à un utilisateur de prendre le dispositif dans la paume de la main,
- plusieurs ensembles de mesure du mouvement et/ou de la force appliquée par un doigt selon une direction d'appui, chaque ensemble de mesure comprenant un capteur de déformation, le capteur de déformation comprenant un corps déformable, chaque ensemble de mesure comprenant également :
   - un palier de guidage, solidaire du corps principal,
   - un axe présentant une surface d'appui, la surface d'appui étant en contact avec le corps déformable,
   - un tube, adapté à coulisser en translation dans le palier de guidage et autour de l'axe selon la direction d'appui, présentant une tête adaptée à fixer le doigt au tube,
   - un premier ressort reliant le palier au tube,
   - un deuxième ressort reliant l'axe au tube,
   le premier ressort et le deuxième ressort étant précontraints, de manière à précontraindre le corps déformable et à maintenir le tube à l'équilibre en l'absence d'appui du doigt sur la tête,
   le premier ressort et le deuxième ressort étant chacun en compression à l'équilibre du système de mesure,
   cet agencement du dispositif permettant de réaliser :
      - des mesures de force de flexion du doigt, par déplacement du doigt vers le corps déformable selon la direction d'appui,
      - des mesures de force d'extension du doigt, par déplacement du doigt à l'opposé du corps déformable selon la direction d'appui.

L'invention est avantageusement complétée par les caractéristiques suivantes, prises individuellement ou en l'une quelconque de leurs combinaisons techniquement possibles :
- l'axe présente la surface d'appui sur son extrémité inférieure, ladite extrémité inférieure étant logée dans une ouverture du corps déformable et une extrémité supérieure située dans le tube au-dessus du palier,
- la surface d'appui est une coupelle tronconique,
- le deuxième ressort est guidé par l'axe et est fixé à un épaulement intérieur du tube,
- le premier ressort est guidé par le tube,
- le premier ressort et le deuxième ressort présentent la même raideur de ressort,
- le dispositif comprend cinq ensembles de mesure pour les cinq doigts de la main (dont le pouce), chaque ensemble de mesure correspondant à un doigt de la main,
- les cinq ensembles de mesure sont alignés dans le corps principal et les directions d'appui des ensembles de mesure sont comprises dans un même plan,
- la tête comprend un premier aimant, le dispositif comprenant un deuxième aimant adapté à être solidaire d'un doigt à être aimanté par le premier aimant,
- les cinq ensembles de mesure sont agencés consécutivement dans le corps principal de manière à correspondre au pouce, à l'index, au majeur, à l'annulaire et à l'auriculaire, et dans lequel, à l'équilibre, les têtes correspondant au majeur et à l'annulaire sont agencées à une distance du corps principal supérieure à la distance du corps principal aux autres têtes,
- les cinq ensembles de mesure sont agencés consécutivement dans le corps principal de manière à correspondre au pouce, à l'index, au majeur, à l'annulaire et à l'auriculaire, et l'angle formé par la direction d'appui de l'ensemble correspondant à l'annulaire et par la direction d'appui de l'ensemble correspondant à l'auriculaire est supérieur à 5° et préférentiellement supérieur à 8°,
- les cinq ensembles de mesure sont agencés consécutivement dans le corps principal de manière à correspondre au pouce, à l'index, au majeur, à l'annulaire et à l'auriculaire, et l'angle formé par la direction d'appui de l'ensemble correspondant au pouce et par la direction d'appui de l'ensemble correspondant à l'index est supérieur à 100° et préférentiellement supérieur à 120°,
- les bords des extrémités de l'axe, du palier, du tube sont arrondis (pour améliorer le guidage du piston et la régularité de la course du tube piston),
- la raideur et la longueur des ressorts sont choisies de sorte à permettre une mesure de force en extension et une mesure de flexion sur une longueur de 10 mm, le débattement maximum du tube dans le corps principal étant de 10 mm,
- le corps déformable est précontraint par une force de traction de -1 N pour permettre une gamme de force mesurable de 6 N, comprise entre -1 N et 5 N, et avec une résolution de 0,1N.
- le dispositif permet de mesurer la force de chaque doigt en flexion dynamique (gamme de 1N) de l'équilibre au débattement maximum, la force de chaque doigt en flexion statique (isométrique, gamme de 4N), lorsque le tube est en appui fixe sur la surface d'appui de l'axe, au débattement maximum ; et/ou la force de chaque doigt en extension dynamique (gamme de 1N) de l'équilibre au débattement minimum,
- le corps principal présente une surface plane sur laquelle sont les quatre ensembles de mesure de l'index, au majeur, à l'annulaire et à l'auriculaire, et une surface biseautée pour l'ensemble de mesure du pouce.

En d'autres termes, les avantages obtenus notamment avec un exemple de mode réalisation qui n'est pas limitatif de toutes les possibilités de réalisation peuvent être les suivants :

### Ergonomie et hygiène

- Le dispositif est portable et peut être maintenu confortablement dans plusieurs postures/positions (par exemple assis avec la main sur la cuisse, allongé au lit la main posée sur une table). Un corps d'appui arrondi dans la paume et un attachement autour de la main (par scratch par exemple) aide à garder la position d'utilisation du dispositif (sans effort volontaire de l'utilisateur).
- Un piston pour le pouce est ajouté
- La position des ensembles de mesure (chaque ensemble de mesure comprenant un piston) est compatible avec une utilisation du dispositif par la main droite et la main gauche. Les têtes des pistons pour les doigts 3 et 4 sont plus hautes que les têtes des autres pistons (+7mm) pour compenser la différence de longueur entre les doigts.
- Les doigts sont fixés, par des aimants, sur les pistons pour garantir un contact en continu avec les capteurs de force. La fixation des doigts est faisable par le sujet seul (sans aide) et la fixation est simple à faire.
- Les surfaces de contact sont faciles à nettoyer avec un désinfectant.

### Mesure de force

- Le dispositif permet de mesurer la force et/ou le déplacement du pouce (doigt 1).
- Le dispositif permet une mesure de force en flexion (10mm) et en extension (10mm). Le débattement total peut être égal à 20 mm.
- Le dispositif permet de mesures la force en dynamique (gamme de 2N) et en statique (isométrique, gamme de 3N). Donc en total : 5N en flexion, et 5N en extension.
- Le signal de force n'est pas perturbé par la transition entre mode dynamique et statique.

### Interface et jeux

Le dispositif peut être utilisé avec les tâches décrites dans la demande de brevet WO2016184935.
- L'affichage visuel des cibles et des forces de l'utilisateur peut se faire en temps-réel et sur un support séparé et portable (tablette, etc.).
- Un retour (en anglais « *feedback* ») de performance par essai peut être prévu (un son qui indique réussite, un autre son pour un échec, au moins pour le suivi, la mémorisation motrice, et la tâche « frappe de plusieurs doigts, en anglais le « *multi-finger tapping* »).
- Le dispositif permet d'établir le niveau de chaque utilisateur. Un test de vitesse de frappes du doigt (activation), en anglais « *finger tapping speed* », sur 15 secondes peut par exemple permettre d'établir la vitesse de frappe, en anglais « *tapping rate* » de l'index. Le résultat donne 10 niveaux pour les jeux (1 à 5 frappes correspondent au niveau 1, 6 à 10 frappes correspondent au niveau 2, et ainsi de suite, jusqu'à plus de 50 frappes qui correspondent au niveau 10). Les niveaux correspondent à la vitesse de déroulement des taches. Par exemple dans la tâche dite de suivi de force, en anglais « *force tracking* », le niveau 1 correspond au même temps d'affichage qu'actuel, le niveau 2 correspond à un temps d'affichage 20% plus rapide que dans le niveau 1, le niveau 3 correspond à un affichage 40% plus rapide que dans le niveau 1, jusqu'au niveau 10 qui correspond à un affichage 200% plus rapide que le niveau 1.

- Quatre jeux sont inclus : le suivi de force, en anglais « *force tracking* » (suivi du trajet de force sur l'écran, en mode flexion-extension), la frappe d'un seul doigt en anglais « *single finger tapping* » (taper dans le rythme avec un feedback visuel et auditif), mémorisation d'une séquence motrice (cinq séquences différentes sont proposées), et la frappe multi-doigts en anglais « *multifinger tapping* » (taper une combinaison des doigts selon des configurations affichées).
- Le dispositif permet une quantification automatique des mesures de la performance, transmise par l'affichage d'un score après chaque tache (et calculé selon l'erreur et le temps de relâchement ; la fréquence maximale et la variation du timing ; le pourcentage de réussite en phase d'apprentissage et la mémorisation ; le pourcentage de réussite et l'indépendance des doigts).

### PRESENTATION DES DESSINS

D'autres caractéristiques et avantages ressortiront encore de la description qui suit, laquelle est purement illustrative et non limitative, et doit être lue en regard des figures annexées, parmi lesquelles :
- la figure 1 est une coupe d'un dispositif comprenant cinq ensembles de mesure,
- la figure 2 illustre un ensemble de mesure,
- la figure 3, la figure 4 et la figure 5 illustrent le même ensemble de mesure dans des configurations correspondant respectivement à l'équilibre, à la flexion d'un doigt et à l'extension d'un doigt,
- la figure 6 illustre une vue agrandie d'une partie d'un ensemble de mesure comprenant un pallier, un tube, et un premier ressort,
- la figure 7 illustre une partie d'un ensemble de mesure comprenant un tube et un axe, l'axe présentant une extrémité arrondie.

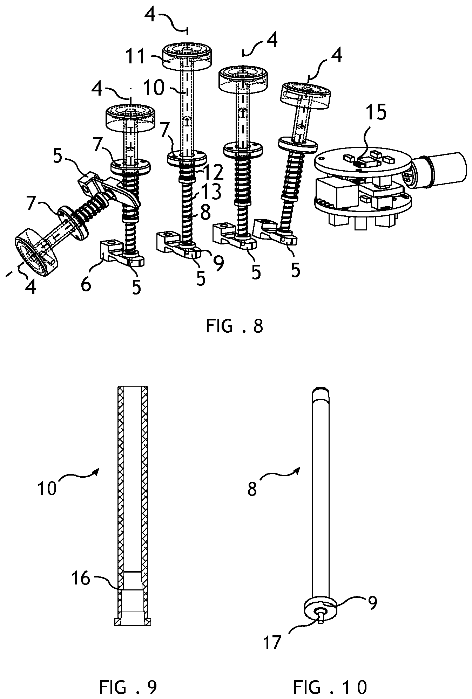

- la figure 8 illustre un agencement de cinq ensembles de mesure,
- la figure 9 est une vue en coupe du tube d'un ensemble de mesure, le tube présentant un épaulement interne,
- la figure 10 illustre un axe présentant une saillie destinée à venir s'encastrer dans un ajour dans la surface d'un corps déformable.

### DESCRIPTION DETAILLEE DE L'INVENTION

### Description générale du dispositif 1

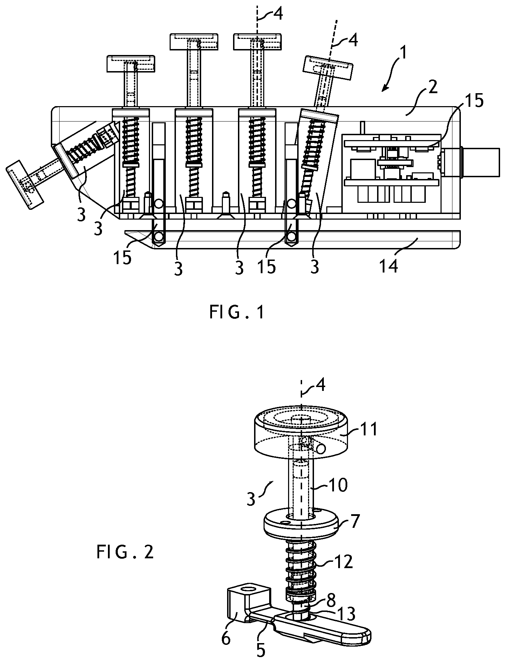

En référence à la figure 1, le dispositif 1 comprend un corps principal 2. Les dimensions du corps principal 2 permettent à un utilisateur de prendre le dispositif 1 dans la paume de la main (main gauche ou main droite). Le corps principal 2 peut comprendre un corps d'appui 14 pouvant être tenu dans la paume de la main. Le corps d'appui 14 se présente sous la forme d'une plaque. Le corps d'appui 14 est relié mécaniquement au corps principal 2 par quatre bras 15, qui peuvent être rendus à la fois solidaires du corps d'appui 14 et du corps principal 2. Le corps d'appui 14 peut, dans une configuration différente, être déplacé par un glissement des bras 15 dans le corps principal 2 afin d'adapter la taille dispositif 1 à la paume de la main de l'utilisateur. Préférentiellement, le corps d'appui 14 peut présenter une forme arrondie destinée à être placée contre la paume de la main de l'utilisateur. Le corps principal 2 peut également préférentiellement comprendre des moyens d'attachement du dispositif 1 à la main, par exemple une lanière dont la taille est adaptable par une fixation crochets et boucles textile.

Le dispositif 1 présente cinq ensembles de mesure 3, partiellement insérés dans le corps principal 2. Chacun des ensembles de mesure 3 peut correspondre à l'un des doigts de la main. Préférentiellement, quatre ensembles de mesure 3 correspondant à l'index, au majeur, à l'annulaire et à l'auriculaire sont disposés ou insérés au travers de la même surface plane du corps principal 2. Préférentiellement, le corps principal 2 présente également une surface biseautée au travers de laquelle est inséré l'ensemble de mesure 3 correspondant au pouce.

Le dispositif 1 comprend une unité de traitement électronique 15. L'unité de traitement électronique 15 est adaptée à recevoir et à traiter des signaux électriques en provenance des différents systèmes de mesure 3. L'unité de traitement électronique 15 est également adaptée à émettre des signaux représentatifs des signaux électriques en provenance des différents systèmes de mesure 3 vers l'extérieur du dispositif 1, par exemple par voie filaire ou sans fil.

### Description générale d'un système de mesure 3

En référence à la figure 2, un ensemble de mesure 3 comprend un capteur de déformation 6, par exemple une jauge de déformation. Le capteur de déformation 6 comprend un corps déformable 5. Le capteur de déformation 6 est relié à une unité de traitement électronique 15. Le capteur de déformation 6 est adapté à émettre un signal électrique représentatif d'une force appliquée sur le corps déformable 5.

L'ensemble de mesure 3 comprend également un palier de guidage 7. Le palier de guidage 7 est solidaire du corps principal 2.

L'ensemble de mesure 3 comprend un tube 10. Le tube 10 est adapté à coulisser, par exemple en translation, dans le palier de guidage 7, selon une direction d'appui 4.

L'ensemble de mesure 3 comprend un axe 8 (dit « piston-axe »). Le tube 10 (dit « tube-piston ») est au moins ouvert à l'une de ses extrémités, pour permettre la réception de l'axe 8, de manière à former un piston. Le tube 10 est adapté à coulisser autour de l'axe 8 selon la direction d'appui 4. L'axe 8 présente une surface d'appui, non visible dans la figure 2, en contact avec le corps déformable 5, sur laquelle l'axe 8 vient prendre appui de manière à déformer le corps déformable 5 du capteur de force 6.

L'axe 8 est tenu ainsi en position verticale ou inclinée dans le dispositif 1, uniquement par l'appui de la surface d'appui 9 sur le corps déformable 5 par son bout inférieur logé librement dans une ouverture du corps déformable 5, et par son maintien à l'intérieur du tube 10.

L'ensemble de mesure 3 comprend un premier ressort 12. Le premier ressort 12 est relié mécaniquement au palier de guidage 7 et au tube 10. Ainsi, une force de rappel relie le palier de guidage 7 et le tube 10. Par force de rappel, on désigne à la fois une force attractrice et répulsive. La force de rappel est considérée dans son sens algébrique. Préférentiellement, le premier ressort 12 est guidé par le tube 10. Notamment, une première extrémité du ressort 12 peut être reliée à une l'extrémité du tube 10 destinée à être insérée dans le corps principal 2, et une deuxième extrémité du premier ressort 12 peut être reliée au palier de guidage 7.

L'ensemble de mesure 3 comprend un deuxième ressort 12. Le deuxième ressort 13 est reliée mécaniquement à l'axe 8 et au tube 10. Ainsi, une force de rappel relie l'axe 8 et le tube 10. Préférentiellement, le deuxième ressort 13 est guidé par l'axe 8. Notamment, une première extrémité du deuxième ressort 13 peut être reliée à l'extrémité de l'axe 8 présentant la surface d'appui 9, et une deuxième extrémité du deuxième ressort 13 peut être fixée à un épaulement intérieur du tube 10. De manière générale, le premier ressort 12 et le deuxième ressort 13 sont agencés de manière à contraindre le tube 10 dans des sens opposés, et préférentiellement dans deux sens opposés selon la direction d'appui 4.

La configuration du premier ressort 12 et du deuxième ressort 13 permet de déplacer la surface d'appui 9 dans un premier sens selon la direction d'appui 4 par rapport à une position d'équilibre de la surface d'appui 9 lors d'une pression sur le tube 10, et de déplacer la surface d'appui 9 dans un second sens selon la direction d'appui 4 par rapport à la position d'équilibre de la surface d'appui 9 lors d'une traction du tube 10. Ainsi, cet agencement du dispositif 1 permet de réaliser des mesures de force de flexion du doigt, par déplacement du doigt vers le corps déformable selon la direction d'appui 4, et des mesures de force d'extension du doigt, par déplacement du doigt à l'opposé du corps déformable 5 selon la direction d'appui 4.

De manière alternative, il est possible d'inverser dans le mécanisme le tube 10 et l'axe 8.

Le dispositif de mesure 3 comprend également une tête 11, solidaire du tube 10. La tête 11 est adaptée à poser le doigt sur le tube 10 et/ou à fixer le doigt sur le tube 10. En particulier, la tête 11 peut présenter une face adaptée au contact avec le doigt. La tête 11 comprend préférentiellement un premier aimant, le dispositif 1 comprenant un deuxième aimant adapté à être solidaire d'un doigt et à être aimanté par le premier aimant. Ainsi, l'ensemble solidaire formé par la tête 11 et le tube 10 peut suivre le mouvement ou les contraintes du doigt en flexion et en extension. D'autres moyens de fixation peuvent être utilisés, tels que des lanières ou des encoches.

### Fonctionnement d'un système de mesure 3

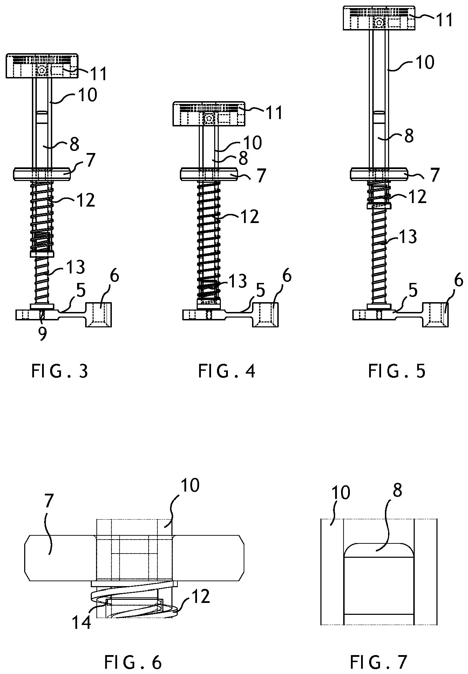

La figure 3, la figure 4 et la figure 5 illustrent respectivement des configurations du système de mesure 3 correspondant à l'équilibre, à la flexion d'un doigt et à l'extension d'un doigt. Par élément « précontraint », on entend que l'élément subit des contraintes mécaniques dans la position d'équilibre du dispositif 1, sans action d'un utilisateur, entraînant potentiellement une déformation de l'élément.

En référence à la figure 3, le premier ressort 12 et le deuxième ressort 13 sont préférentiellement précontraints, c'est-à-dire que le premier ressort 12 et le deuxième ressort 13 sont compressés à la position d'équilibre du dispositif 1, quand aucune force extérieure au dispositif 1 n'est exercée sur le système de mesure 3, en particulier sur le tube 10. La précontrainte du premier ressort 12 et du deuxième ressort 13 est réalisée de telle manière que le corps déformable 5 est également précontraint dans la position d'équilibre du dispositif 1. Ainsi, le corps déformable 5 est déformé à l'équilibre, ce qui lui permet de suivre l'axe 8 lors d'un mouvement d'extension, et ainsi au capteur de déformation 6 de détecter l'extension d'un doigt. Préférentiellement, le corps déformable 5 est précontraint par une force de traction égale à -1 N (c'est-à-dire une force dirigée de l'axe 8 vers le corps déformable 5) pour permettre une gamme de force mesurable de 6 N, comprise entre -1 N et 5 N, et avec une résolution de 0,1 N.

La précontrainte du premier ressort 12 et du deuxième ressort 13 permet également de maintenir le tube 10 à l'équilibre en l'absence d'appui du doigt sur la tête 11. La précontrainte du premier ressort 12 et du deuxième ressort 13 permet enfin d'assurer une linéarité optimale dans la relation entre la force exercée par le doigt au système de mesure 3 et la force exercée par l'axe 8 sur le capteur de force 6.

L'axe 8 présente une surface d'appui 9. La surface d'appui 9 est présentée par une extrémité de l'axe 8 qui n'est pas couverte par le tube 10. La surface d'appui 9 peut être par exemple plane, conique, ou formée par une protubérance de l'axe 8. Le corps déformable 5 présente préférentiellement une surface complémentaire à la surface d'appui 9. Ainsi, il est possible d'assurer un contact précis entre l'axe 8 et le corps déformable 5, en réduisant les jeux mécaniques et ainsi en augmentant la précision des mesures réalisées par le dispositif 1. L'axe 8 peut par exemple présenter une surface d'appui 4 en coupelle tronconique, présentant préférentiellement une saillie 17 destinée à venir s'encastrer dans un ajour dans la surface du corps déformable 5. L'axe 8 présente la surface d'appui 9 sur son extrémité inférieure logée dans une ouverture du corps déformable 5 et une extrémité supérieure située dans le tube au-dessus du palier. Il est ainsi possible d'éviter tout jeu dans des directions différentes de la direction d'appui 4.

Préférentiellement, le premier ressort 12 et le deuxième ressort 13 présentent la même raideur. Ainsi, la flexion et l'extension d'un doigt peuvent être déterminée avec la même sensibilité par le dispositif 1.

Préférentiellement, le premier ressort 12 et/ou le deuxième ressort 13 présente une raideur comprise entre 0,01 N/mm et 1 N/mm. Ainsi, la raideur du ou des ressorts est adaptée à la force d'un doigt.

En référence à la figure 4, un doigt en flexion peut exercer une contrainte sur la tête 11 du système de mesure 3. Le tube 10 peut ainsi coulisser autour de l'axe 8, jusqu'à ce que l'extrémité fermée du tube 10 soit en contact avec l'axe 8. Dans cette configuration, le premier ressort 12 est moins compressé que dans la position d'équilibre. Le premier ressort 12 est, dans cette configuration, toujours compressé par rapport à la position d'équilibre du premier ressort 12 seul, c'est-à-dire considéré séparément du dispositif, sans contrainte extérieure. Dans cette configuration, le deuxième ressort 13 est plus compressé que dans la position d'équilibre. Le tube 10 peut présenter un épaulement interne 16, agencé à une distance de l'extrémité du tube 10 opposée à la tête 11, correspondant à la longueur maximale de compression du deuxième ressort 13. Le deuxième ressort 13 peut ainsi être compressé entre l'épaulement interne 16 et l'extrémité de l'axe 8 en contact avec le corps déformable 5 selon la distance définie par l'épaulement interne 16 quand l'extrémité du tube 10 opposée à la tête 11 vient rejoindre l'extrémité de l'axe 8. Dans cette configuration, le corps déformable 5 est plus déformé que dans la configuration à l'équilibre illustrée dans la figure 3.

En référence à la figure 5, un doigt en extension, relié à la tête 11, peut exercer une contrainte sur la tête 11. Le tube 10 peut ainsi coulisser autour de l'axe 8 pour s'en écarter. Dans cette configuration, le premier ressort 12 est plus comprimé que dans la position d'équilibre de l'ensemble de mesure 3. Le deuxième ressort 13 est moins comprimé que dans la position d'équilibre de l'ensemble de mesure 13, et préférentiellement plus comprimé que dans la position d'équilibre du deuxième ressort 13 seul, c'est-à-dire considéré séparément du dispositif, sans contrainte extérieure. Dans cette configuration, le corps déformable est moins contraint, et ainsi moins déformé, que dans la position d'équilibre de l'ensemble de mesure 3. Ainsi, il est possible de mesurer l'extension d'un doigt.

Préférentiellement, les raideurs du premier ressort 12, du deuxième ressort 13, ainsi que les dimensions du tube 10 et de l'axe 8 sont choisie de manière à permettre un débattement du tube 10 par rapport au corps principal 2 de 10 mm par rapport à une position d'équilibre du tube 8. Ce débattement peut rapprocher ou éloigner le tube 10 du corps principal 2 selon que le doigt est respectivement en flexion ou en extension.

En référence à la figure 6 et à la figure 7, les bords des différentes extrémités des pièces du dispositif 1, préférentiellement de l'axe 8, du tube 10 et/ou du palier 7 sont arrondis. Par « arrondi », on entend que les bords présentent un rayon de courbure supérieur à 50 µm, préférentiellement supérieur à 100 µm et notamment supérieur à 150 µm. Ainsi, le frottement entre les différentes pièces, par exemple entre l'axe 8 et le tube 10, est diminué lors du mouvement du système de mesure 3. La figure 6 illustre des bords de palier de guidage 7 arrondis et un épaulement 14 arrondi dans le tube 10. La figure 7 illustre une extrémité de l'axe 8 arrondie.

### Agencement des ensembles de mesure 3

En référence à la figure 6, l'agencement particulier des différents ensembles de mesure 3 dans le corps principal 2 permet de résoudre des problèmes de l'art antérieur.

Les directions d'appui 4 des ensembles de mesure 3, notamment des 35 cinq ensembles de mesure 3, sont comprises dans un même plan. Ainsi, les ensembles de mesure 3 sont symétriques par rapport à un plan, ce qui permet au dispositif d'être adapté à une main gauche et à une main droite.

Les têtes 11 correspondant au majeur et à l'annulaire sont agencées à une distance du corps principal 2 supérieure à la distance des autres têtes 11 au corps principal 2.

L'angle formé par la direction d'appui 4 de l'ensemble de mesure 3 correspondant à l'annulaire et par la direction d'appui 4 de l'ensemble de mesure 3 correspondant à l'auriculaire est préférentiellement supérieur à 5° et notamment supérieur à 8°. De plus, l'angle formé par la direction d'appui 4 de l'ensemble de mesure 3 correspondant au pouce et par la direction d'appui 4 de l'ensemble de mesure 3 correspondant à l'index est supérieur à 100° et préférentiellement supérieur à 120°.

Ainsi, les distances entre les têtes 11 des ensembles de mesure 3 en positions d'équilibre correspondent aux distances entre les différentes 15 extrémités des doigts au repos dans une position de pronation.

## Revendications

1. Dispositif (1) pour quantifier la dextérité des doigts d'une main, comprenant :
- un corps principal (2), les dimensions du corps principal permettant à un utilisateur de prendre le dispositif dans la paume de la main,
- plusieurs ensembles de mesure (3) du mouvement et/ou de la force appliquée par un doigt selon une direction d'appui (4), chaque ensemble de mesure (3) comprenant un capteur de déformation (6), le capteur de déformation comprenant un corps déformable (5), chaque ensemble de mesure comprenant également :
• un palier de guidage (7), solidaire du corps principal,
• un axe (8) présentant une surface d'appui (9), la surface d'appui étant en contact avec le corps déformable (5),
• un tube (10), adapté à coulisser en translation dans le palier de guidage et autour de l'axe (8) selon la direction d'appui (4), présentant une tête (11) adaptée à fixer le doigt au tube,
• un premier ressort (12) reliant le palier (7) au tube (10),
• un deuxième ressort (13) reliant l'axe (8) au tube (10),
le premier ressort (12) et le deuxième ressort (13) étant précontraints, de manière à précontraindre le corps déformable (5) et à maintenir le tube (10) à l'équilibre en l'absence d'appui du doigt sur la tête (11),
le premier ressort (12) et le deuxième ressort (13) étant chacun en compression à l'équilibre du système de mesure (3),
cet agencement du dispositif permettant de réaliser :
- des mesures de force de flexion du doigt, par déplacement du doigt vers le corps déformable (5) selon la direction d'appui (4),
- des mesures de force d'extension du doigt, par déplacement du doigt à l'opposé du corps déformable (5) selon la direction d'appui (4).

2. Dispositif selon la revendication 1, dans lequel l'axe (8) présente la surface d'appui (9) sur son extrémité inférieure, ladite extrémité inférieure étant logée dans une ouverture du corps déformable et une extrémité supérieure située dans le tube (10) au-dessus du palier (7).

3. Dispositif selon la revendication 1 ou 2, dans lequel la surface d'appui (9) est une coupelle tronconique.

4. Dispositif selon l'une des revendications 1 à 3, dans lequel le deuxième ressort (13) est guidé par l'axe (8) et est fixé à un épaulement intérieur du tube (10).

5. Dispositif selon l'une des revendications 1 à 4, dans lequel le premier ressort (12) est guidé par le tube (10).

6. Dispositif selon l'une des revendications 1 à 5, dans lequel le premier ressort (12) et le deuxième ressort (13) présentent la même raideur de ressort.

7. Dispositif selon l'une des revendications 1 à 6, comprenant cinq ensembles de mesure (3) pour les cinq doigts de la main, chaque ensemble de mesure (3) correspondant à un doigt de la main.

8. Dispositif selon l'une des revendications 1 à 7, dans lequel les directions d'appui (4) des ensembles de mesure (3) sont comprises dans un même plan.

9. Dispositif selon l'une des revendications 1 à 8, dans lequel la tête (11) comprend un premier aimant, le dispositif comprenant un deuxième aimant adapté à être solidaire d'un doigt à être aimanté par le premier aimant.

10. Dispositif selon l'une des revendications 1 à 9, dans lequel les cinq ensembles de mesure (3) sont agencés consécutivement dans le corps principal (2) de manière à correspondre au pouce, à l'index, au majeur, à l'annulaire et à l'auriculaire, et dans lequel, à l'équilibre, les têtes (11) correspondant au majeur et à l'annulaire sont agencées à une distance du corps principal (2) supérieure à la distance du corps principal aux autres têtes (11).

11. Dispositif selon l'une des revendications 1 à 10, dans lequel les cinq ensembles de mesure sont agencés consécutivement dans le corps principal de manière à correspondre au pouce, à l'index, au majeur, à l'annulaire et à 35 l'auriculaire, et dans lequel :
- l'angle formé par la direction d'appui de l'ensemble correspondant à l'annulaire et par la direction d'appui de l'ensemble correspondant à l'auriculaire est supérieur à 5° et préférentiellement supérieur à 8°.

12. Dispositif selon l'une des revendications 1 à 11, dans lequel les cinq ensembles de mesure sont agencés consécutivement dans le corps principal (2) de manière à correspondre au pouce, à l'index, au majeur, à l'annulaire et à l'auriculaire, et dans lequel :
- l'angle formé par la direction d'appui de l'ensemble correspondant au pouce et par la direction d'appui de l'ensemble correspondant à l'index est supérieur à 100° et préférentiellement supérieur à 120°.

13. Dispositif selon l'une des revendications 1 à 12, dans lequel les bords des extrémités de l'axe (8), du palier (7), du tube (10) sont arrondis (pour améliorer le guidage du piston et la régularité de la course du tube piston).

14. Dispositif selon l'une des revendications 1 à 13, dans lequel la raideur et la longueur des ressorts (12, 13) sont choisies de sorte à permettre une mesure de force en extension et une mesure de flexion sur une longueur de 10 mm, le débattement maximum du tube dans le corps principal étant de 10mm.

15. Dispositif selon l'une des revendications 1 à 14 dans lequel le corps déformable (5) est précontraint par une force de traction de -1N pour permettre une gamme de force mesurable de 6N, comprise entre -1 N et 5 N, et avec une résolution de 0,1 N.

16. Dispositif selon l'une des revendications 1 à 15, dans lequel le dispositif permet de mesurer :
- la force de chaque doigt en flexion dynamique (range de 1 N) de l'équilibre au débattement maximum;
- la force de chaque doigt en flexion statique (isométrique, range de 4N), lorsque le tube est en appui fixe sur la surface d'appui de l'axe, au débattement maximum; et/ou
- la force de chaque doigt en extension dynamique (range de 1N) de l'équilibre au débattement minimum.

17. Dispositif selon l'une des revendications 1 à 16, dans lequel le corps principal présente :
- une surface plane sur laquelle sont les quatre ensembles de mesure de l'index, au majeur, à l'annulaire et à l'auriculaire, et
- une surface biseautée pour l'ensemble de mesure du pouce.

## Patentansprüche

1. Vorrichtung (1) zur Quantifizierung der Fingerfertigkeit einer Hand, umfassend:
- einen Hauptkörper (2), wobei die Abmessungen des Hauptkörpers es einem Benutzer ermöglichen, die Vorrichtung in der Handfläche zu halten;
- mehrere Anordnungen (3) zum Messen der Bewegung und/oder der Kraft, die von einem Finger entlang einer Auflagerichtung (4) ausgeübt wird, wobei jede Messanordnung (3) einen Verformungssensor (6) umfasst, wobei der Verformungssensor einen verformbaren Körper (5) umfasst,
wobei jede Messanordnung außerdem umfasst:
• ein mit dem Hauptkörper fest verbundenes Führungslager (7),
• einen Stift (8) mit einer Auflagefläche (9), wobei die Auflagefläche mit dem verformbaren Körper (5) Kontakt hat,
• ein Rohr (10), das dafür ausgelegt ist, translatorisch in dem Führungslager und um den Stift (8) in der Auflagerichtung (4) zu gleiten, und das einen Kopf (11) aufweist, der dafür ausgelegt ist, den Finger an dem Rohr zu fixieren,
• eine erste Feder (12), die das Lager (7) mit dem Rohr (10) verbindet,
• eine zweite Feder (13), die den Stift (8) mit dem Rohr (10) verbindet,
wobei die erste Feder (12) und die zweite Feder (13) vorgespannt sind, um den verformbaren Körper (5) vorzuspannen und das Rohr (10) im Gleichgewicht zu halten, wenn der Finger nicht auf dem Kopf (11) aufliegt;
wobei sich die erste Feder (12) und die zweite Feder (13) jeweils im Gleichgewicht des Messsystems (3) komprimiert sind,
wobei diese Anordnung der Vorrichtung die Durchführung ermöglicht von:
- Messungen der Beugekraft des Fingers, indem sich der Finger entlang der Auflagerichtung (4) in Richtung des verformbaren Körpers (5) bewegt;
- Messungen der Streckkraft des Fingers, indem sich der Finger entlang der Auflagerichtung (4) entgegengesetzt zu dem verformbaren Körper (5) bewegt.

2. Vorrichtung nach Anspruch 1, wobei der Stift (8) die Auflagefläche (9) an seinem unteren Ende aufweist, wobei das untere Ende in einer Öffnung des verformbaren Körpers untergebracht ist und sich ein oberes Ende in dem Rohr (10) oberhalb des Lagers (7) befindet.

3. Vorrichtung nach Anspruch 1 oder 2, wobei die Auflagefläche (9) eine kegelstumpfförmige Schale ist.

4. Vorrichtung nach einem der Ansprüche 1 bis 3, wobei die zweite Feder (13) von dem Stift (8) geführt und an einem inneren Absatz des Rohrs (10) befestigt ist.

5. Vorrichtung nach einem der Ansprüche 1 bis 4, wobei die erste Feder (12) durch das Rohr (10) geführt ist.

6. Vorrichtung nach einem der Ansprüche 1 bis 5, wobei die erste Feder (12) und die zweite Feder (13) die gleiche Federsteifigkeit aufweisen.

7. Vorrichtung nach einem der Ansprüche 1 bis 6, umfassend fünf Messanordnungen (3) für die fünf Finger der Hand, wobei jede Messanordnung (3) einem Finger der Hand entspricht.

8. Vorrichtung nach einem der Ansprüche 1 bis 7, wobei die Auflagerichtungen (4) der Messanordnungen (3) in derselben Ebene liegen.

9. Vorrichtung nach einem der Ansprüche 1 bis 8, wobei der Kopf (11) einen ersten Magneten umfasst, wobei die Vorrichtung einen zweiten Magneten umfasst, der dafür ausgelegt ist, mit einem durch den ersten Magneten zu magnetisierenden Finger fest verbunden zu werden.

10. Vorrichtung nach einem der Ansprüche 1 bis 9, wobei die fünf Messanordnungen (3) hintereinander in dem Hauptkörper (2) so angeordnet sind, dass sie dem Daumen, dem Zeigefinger, dem Mittelfinger, dem Ringfinger und dem kleinen Finger entsprechen, und wobei im Gleichgewicht die dem Mittelfinger und dem Ringfinger entsprechenden Köpfe (11) in einem Abstand von dem Hauptkörper (2) angeordnet sind, der größer ist als der Abstand des Hauptkörpers zu den anderen Köpfen (11).

11. Vorrichtung nach einem der Ansprüche 1 bis 10, wobei die fünf Messanordnungen hintereinander in dem Hauptkörper so angeordnet sind, dass sie dem Daumen, dem Zeigefinger, dem Mittelfinger, dem Ringfinger und dem kleinen Finger entsprechen, und wobei:
- der Winkel, der durch die Auflagerichtung der dem Ringfinger entsprechenden Anordnung und die Auflagerichtung der dem kleinen Finger entsprechenden Anordnung gebildet wird, größer als 5° und vorzugsweise größer als 8° ist.

12. Vorrichtung nach einem der Ansprüche 1 bis 11, wobei die fünf Messanordnungen hintereinander in dem Hauptkörper (2) so angeordnet sind, dass sie dem Daumen, dem Zeigefinger, dem Mittelfinger, dem Ringfinger und dem kleinen Finger entsprechen, und wobei:
- der Winkel, der durch die Auflagerichtung der dem Daumen entsprechenden Anordnung und die Auflagerichtung der dem Zeigefinger entsprechenden Anordnung gebildet wird, größer als 100° und vorzugsweise größer als 120° ist.

13. Vorrichtung nach einem der Ansprüche 1 bis 12, wobei die Kanten der Enden des Stifts (8), des Lagers (7), des Rohrs (10) abgerundet sind (zur Verbesserung der Führung des Kolbens und der Gleichmäßigkeit des Hubs des Kolbenrohrs).

14. Vorrichtung nach einem der Ansprüche 1 bis 13, wobei die Steifigkeit und die Länge der Federn (12, 13) so gewählt sind, dass sie eine Messung der Streckkraft und eine Messung der Beugekraft über eine Länge von 10 mm ermöglichen, wobei der maximale Weg des Rohrs in dem Hauptkörper 10 mm beträgt.

15. Vorrichtung nach einem der Ansprüche 1 bis 14, wobei der verformbare Körper (5) mit einer Zugkraft von -1 N vorgespannt ist, um einen messbaren Kraftbereich von 6 N, zwischen -1 N und 5 N, und mit einer Auflösung von 0,1 N zu ermöglichen.

16. Vorrichtung nach einem der Ansprüche 1 bis 15, wobei es die Vorrichtung ermöglicht zu messen:
- die Kraft jedes Fingers bei dynamischer Beugung (Bereich von 1 N) vom Gleichgewicht bis zum maximalen Weg;
- die Kraft jedes Fingers bei statischer Beugung (isometrisch, Bereich von 4 N), wenn das Rohr fest auf der Auflagefläche des Stiftes aufliegt, bei maximalem Weg; und/oder
- die Kraft jedes Fingers bei dynamischer Streckung (Bereich von 1 N) vom Gleichgewicht bis zum minimalen Weg.

17. Vorrichtung nach einem der Ansprüche 1 bis 16, wobei der Hauptkörper aufweist:
- eine ebene Oberfläche, auf der sich die vier Messanordnungen für den Zeigefinger, den Mittelfinger, den Ringfinger und den kleinen Finger befinden, und
- eine abgeschrägte Oberfläche für die Messanordnung für den Daumen.

## Claims

1. A device (1) for quantifying the dexterity of the fingers of a hand, comprising:
- a main body (2), the dimensions of the main body allowing a user to take the device in the palm of the hand,
- several assemblies for measuring (3) the movement and/or the force applied by a finger along a supporting direction (4), each measuring assembly (3) comprising a deformation sensor (6), the deformation sensor comprising a deformable body (5),
each measuring assembly also comprising:
• a guide bearing (7), secured to the main body,
• an axis (8) having a supporting surface (9), the supporting surface being in contact with the deformable body (5),
• a tube (10), adapted to slide in translation in the guide bearing and about the axis (8) along the supporting direction (4), having a head (11) adapted to fix the finger to the tube,
• a first spring (12) connecting the bearing (7) to the tube (10),
• a second spring (13) connecting the axis (8) to the tube (10),
the first spring (12) and the second spring (13) being pre-stressed so as to pre-stress the deformable body (5) and keep the tube (10) at equilibrium in the absence of pressure of the finger on the head (11),
the first spring (12) and the second spring (13) each being in compression at equilibrium of the measuring system (3),
this arrangement of the device making it possible to carry out:
- finger flexion force measurements, by movement of the finger towards the deformable body (5) along the supporting direction (4),
- finger extension force measurements, by movement of the finger away from the deformable body (5) along the supporting direction (4).

2. The device according to claim 1, wherein the axis (8) has the supporting surface (9) on its lower end, said lower end being housed in an opening of the deformable body and an upper end located in the tube (10) above the bearing (7).

3. The device according to claim 1 or 2, wherein the supporting surface (9) is a frustoconical retainer.

4. The device according to any of claims 1 to 3, wherein the second spring (13) is guided by the axis (8) and is fixed to an internal shoulder of the tube (10).

5. The device according to any of claims 1 to 4, wherein the first spring (12) is guided by the tube (10).

6. The device according to any of claims 1 to 5, wherein the first spring (12) and the second spring (13) have the same spring stiffness.

7. The device according to any of claims 1 to 6, comprising five measuring assemblies (3) for the five fingers of the hand, each measuring assembly (3) corresponding to one finger of the hand.

8. The device according to any of claims 1 to 7, wherein the supporting directions (4) of the measuring assemblies (3) are comprised in the same plane.

9. The device according to any of claims 1 to 8, wherein the head (11) comprises a first magnet, the device comprising a second magnet adapted to be secured to a finger to be magnetized by the first magnet.

10. The device according to any of claims 1 to 9, wherein the five measuring assemblies (3) are arranged consecutively in the main body (2) so as to correspond to the thumb, the index finger, the middle finger, the ring finger and the little finger, and wherein, at equilibrium, the heads (11) corresponding to the middle finger and to the ring finger are arranged at a distance from the main body (2) greater than the distance of the main body from the others heads (11).

11. The device according to any of claims 1 to 10, wherein the five measuring assemblies are arranged consecutively in the main body so as to correspond to the thumb, the index finger, the middle finger, the ring finger and the little finger, and wherein:
- the angle formed by the supporting direction of the assembly corresponding to the ring finger and by the supporting direction of the assembly corresponding to the little finger is greater than 5° and preferably greater than 8°.

12. The device according to any of claims 1 to 11, wherein the five measuring assemblies are arranged consecutively in the main body (2) so as to correspond to the thumb, the index finger, the middle finger, the ring finger and the little finger, and wherein:
- the angle formed by the supporting direction of the assembly corresponding to the thumb and by the supporting direction of the assembly corresponding to the index finger is greater than 100° and preferably greater than 120°.

13. The device according to any of claims 1 to 12, wherein the edges of the ends of the axis (8), the bearing (7), the tube (10) are rounded (to improve the guidance of the piston and the regularity of the stroke of the piston tube).

14. The device according to any of claims 1 to 13, wherein the stiffness and the length of the springs (12, 13) are chosen so as to allow an extension force measurement and a flexion measurement over a length of 10 mm, the maximum displacement of the tube in the main body being of 10 mm.

15. The device according to any of claims 1 to 14 wherein the deformable body (5) is pre-stressed by a tensile force of -1N to allow a measurable force range of 6N, comprised between -1N and 5N, and with a resolution of 0.1N.

16. The device according to any of claims 1 to 15, wherein the device allows measuring:
- the force of each finger in dynamic flexion (range of 1N) from equilibrium to maximum displacement;
- the force of each finger in static flexion (isometric, range of 4N), when the tube is in fixed support on the supporting surface of the axis, at maximum displacement; and/or
- the force of each finger in dynamic extension (range of 1N) from equilibrium to minimum displacement.

17. The device according to any of claims 1 to 16, wherein the main body has:
- a planar surface on which the four assemblies for measuring the index finger, the middle finger, the ring finger and the little finger are located, and
- a beveled surface for the thumb measuring assembly.
